# EUROPÄISCHE PATENTANMELDUNG

(11) **EP 1 288 309 A1**
(43) Veröffentlichungstag der Anmeldung: **05.03.2003**
(21) Anmeldenummer: 01120908.7
(22) Anmeldetag: 30.08.2001
(51) Int. Cl.: C12Q 1/68

(54) **Verfahren zur Markierung von L-Nukleinsäuren**

(71) Anmelder: Noxxon Pharma AG, 13355 Berlin (DE)
(72) Erfinder: Frauendorf, Christian, Dr., 13189 Berlin (DE); Hausch, Felix, Dr., Standford, CA 96 (US); Klussmann, Sven, Dr., 10709 Berlin (DE); Lichte, Andrea, Dr., 35037 Marburg (DE)
(74) Vertreter: Bohmann, Armin K., Dr.

(57) **Zusammenfassung**

Die vorliegende Erfindung betrifft ein Verfahren zum Markieren von L-Nukleinsäuren umfassend die Schritte
a) Bereitstellen eines ersten Teils der zu markierenden L-Nukleinsäure,
b) Markieren des ersten Teils mit einem Marker,
c) Bereitstellen eines zweiten Teils der zu markierenden L-Nukleinsäure, und
d) Ligation des in Schritt b) markierten ersten Teils mit dem zweiten Teil der zu markierenden Nukleinsäure.

## Beschreibung

Die vorliegende Erfindung betrifft Verfahren zum Markieren von L-Nukleinsäuren, eine Markierung umfassende L-Nukleinsäuren sowie deren Verwendung.

Die Markierung von chemischen Verbindungen ist eine wesentliche Voraussetzung für deren Anwendung in der Analytik, Diagnostik und im therapeutischen Bereich, insbesondere im Bereich der präklinischen Analytik. Insbesondere bei polymeren Verbindungen eröffnen sich Möglichkeiten der Markierung, die bei kleineren Molekülen häufig deshalb nicht möglich sind, weil durch die Markierung die chemischen, physikalischen oder biologischen Eigenschaften maßgeblich beeinflusst werden würden. Derartige polymere Verbindungen sind Peptide, Proteine, aber auch funktionale Nukleinsäuren, wie Antisense-Oligonukleotide, Ribozyme und Aptamere. Letztere zeichnen sich durch ihre spezifische Bindung an ein Zielmolekül aus. Verfahren zur Herstellung derartiger Aptamere sind beispielsweise beschrieben in der europäischen Patentanmeldung EP 0 533 838.

Eine weitere Klasse von polymeren Verbindungen, die spezifisch an ein Zielmolekül binden, sind die sogenannten Spiegelmere, deren Herstellung beispielsweise in der internationalen Patentanmeldung WO 98/08856 beschrieben ist. Rein chemisch betrachtet sind Spiegelmere L-Oligonukleotide, die von natürlichen Enzymen infolge ihres Aufbaus aus L-Nukleotiden praktisch nicht abgebaut werden können. Neben der Zielmolekül-Spezifität qualifiziert sie diese Eigenschaft zur Anwendung im Bereich Analyse biologischer Proben, Diagnostik und Therapie. Die Markierung von Spiegelmeren ist beispielsweise auch in der präklinischen Analytik erforderlich zur Bestimmung der Pharmakokinetik und Pharmakodynamik dieser neuen Klasse von pharmazeutischen Wirkstoffen.

Die Markierung von polymeren Verbindungen, die aus in biologischen Systemen vorkommenden Komponenten bestehen, kann unter anderem durch verschiedene Enzyme erfolgen. So können natürliche, d.h. D-Nukleinsäuren enzymatisch beispielsweise unter Verwendung einer Kinase 5'-terminal mit ³²P markiert werden. (Sambrook, Fritsch, Maniatis, Molecular Cloning- A laboratory Manual, 2^{nd} Ed. 1989, Cold Spring Harbor Laboratory Press, p.5.68).

Mit der zunehmenden Nutzung von L-Nukleinsäuren in verschiedenen Bereichen besteht ein Bedarf, markierte, insbesondere intern markierte L-Nukleinsäuren bereitzustellen.

Der vorliegenden Erfindung liegt somit die Aufgabe zugrunde, stabil markierte L-Nukleinsäuren bereitzustellen. Eine weitere der vorliegenden Erfindung zugrundeliegende Aufgabe besteht darin, ein Verfahren zur Herstellung derartiger stabil markierter L-Nukleinsäuren bereitzustellen.

Erfindungsgemäß wird die Aufgabe gelöst durch ein Verfahren zum Markieren von L-Nukleinsäuren umfassend die Schritte
a) Bereitstellen eines ersten Teils der zu markierenden L-Nukleinsäure,
b) Markieren des ersten Teils mit einem Marker,
c) Bereitstellen eines zweiten Teils der zu markierenden L-Nukleinsäure, und
d) Ligation des in Schritt b) markierten ersten Teils mit dem zweiten Teil der zu markierenden Nukleinsäure.

In einer Ausführungsform ist vorgesehen, dass der Marker ausgewählt ist aus der Gruppe, die ³H, ¹³C, ¹⁴C, ¹⁵N, ¹⁸O, ³²P, ³³P und ³⁵S umfasst.

In einer weiteren Ausführungsform ist vorgesehen, dass das Markieren des ersten Teils mit einem Marker am 5'-Ende des ersten Teils erfolgt.

In einer noch weiteren Ausführungsform ist vorgesehen, dass das Markieren durch Umsetzen des ersten Teils mit einer Kinase und einem einen Marker tragenden Nukleotid erfolgt.

In einer bevorzugten Ausführungsform ist vorgesehen, dass die Kinase T⁴-Polynukleotid-Kinase ist.

In einer weiteren bevorzugten Ausführungsform ist vorgesehen, dass das Nukleotid ausgewählt ist aus der Gruppe, die γ-[¹⁸O]-ATP, γ-[³²P]-ATP, γ-[³³P]-ATP und γ-[³⁵S]-ATP und Adenosinphosphonate und Derivate der Adenosinphosphonate umfasst.

In einer Ausführungsform ist vorgesehen, dass die Ligation durch Verknüpfen des 5'-Endes des ersten Teils mit dem 3'-Ende des zweiten Teils erfolgt.

In einer weiteren Ausführungsform ist vorgesehen, dass die Ligation der beiden Teile an einer komplementären Matrix erfolgt.

In einer weiteren Ausführungsform ist vorgesehen, dass die Matrix aus L-Desoxyribonukleotiden oder L-Ribonukleotiden besteht.

In einer bevorzugten Ausführungsform ist vorgesehen, dass die komplementäre Matrix aus L-Desoxyribonukleotiden besteht und mindestens ein Ribonukleotid enthält.

In einer noch bevorzugteren Ausführungsform ist vorgesehen, dass nach der Ligation die komplementäre Matrix an dem mindestens einem Ribonukleotid hydrolysiert wird.

In einer Ausführungsform ist vorgesehen, dass das 5'-terminale Nukleotid des ersten Teils G oder C ist.

In einer weiteren Ausführungsform ist vorgesehen, dass das 3'-terminale Nukleotid des zweiten Teils G oder C ist.

In einer noch weiteren Ausführungsform ist vorgesehen, dass die Ligation unter Verwendung eines Kopplungsreagenz erfolgt, wobei das Kopplungsreagenz ausgewählt ist aus der Gruppe, die BrCN und BrCN in Verbindung mit Imidazol bzw. Imidazolderivaten wie beispielsweise Iminodiimidazol oder N-Cyanoimidazol umfassst.

In einer bevorzugten Ausführungsform ist vorgesehen, dass das Kopplungsreagenz eine Mischung aus BrCN, divalenten Metallionen, bevorzugterweise Ni²⁺, Co²⁺, Mn²⁺, Mg²⁺, Cu²⁺, Zn²⁺, und Imidazol, oder ein Carbodiimid ist.

In einer noch bevorzugteren Ausführungsform ist vorgesehen, dass das Carbodiimid EDC (N'-Dimethylaminopropyl-N-ethylcarbodiimid) ist.

In einer Ausführungsform ist vorgesehen, dass die L-Nukleinsäure aus L-Desoxyribonukleotiden besteht. In einer alternativen Ausführungsform ist vorgesehen, dass die L-Nukleinsäure aus L-Ribonukleotiden besteht.

Schließlich ist es auch im Rahmen der vorliegenden Erfindung, dass die L-Nukleinsäure sowohl L-Desoxy- als auch L-Ribonukleotide umfasst.

In einem weiteren Aspekt wird die Aufgabe gelöst durch eine L-Nukleinsäure umfassend eine Markierung, wobei die Markierung an einem nicht-terminalen Nukleotid der Nukleinsäure angeordnet ist.

In einer Ausführungsform ist vorgesehen, dass die Markierung eine radioaktive Markierung ist.

In einer bevorzugten Ausführungsform ist vorgesehen, dass die Markierung ausgewählt ist aus der Gruppe, die ³H, ¹³C, ¹⁴C, ¹⁵N, ¹⁸O, ³²P, ³³P und ³⁵S umfasst.

In einer weiteren Ausführungsform ist vorgesehen, dass zwischen zwei aufeinanderfolgenden Guanidin-Nukleosiden ein radioaktives Nuklid als Marker angeordnet ist.

In einer bevorzugten Ausführungsform ist vorgesehen, dass die Nukleinsäure eine Sequenz gemäß SEQ ID NO:8 aufweist.

In einer besonders bevorzugten Ausführungsform ist vorgesehen, dass das Phosphat zwischen Position 30 und 31 von SEQ ID NO: 8 ³²P ist.

In einem weiteren Aspekt betrifft die vorliegenden Erfindung die Verwendung einer T4-Polynukleotidkinase zum Markieren einer L-Nukleinsäure.

In einer bevorzugten Ausführungsform ist vorgesehen, dass die Markierung innerhalb der L-Nukleinsäure erfolgt.

In einer alternativen Ausführungsform ist vorgesehen, dass die Markierung terminal an der L-Nukleinsäure erfolgt.

In einer Ausführungsform ist dabei vorgesehen, dass die L-Nukleinsäure vollständig aus L-Nukleotiden besteht.

In einer Ausführungsform der erfindungsgemäßen L-Nukleinsäure ist vorgesehen, dass die L-Nukleinsäure aus L-Desoxyribonukleotiden besteht. In einer alternativen Ausführungsform ist vorgesehen, dass die L-Nukleinsäure aus L-Ribonukleotiden besteht. Schließlich ist es auch im Rahmen der vorliegenden Erfindung, dass die L-Nukleinsäure sowohl L-Desoxy- als auch L-Ribonukleotide umfasst.

In einem weiteren Aspekt betrifft die vorliegenden Erfindung die Verwendung der erfindungsgemäßen Nukleinsäuren zum Nachweis einer Verbindung, an die die Nukleinsäure bindet.

Dabei ist in einer bevorzugten Ausführungsform vorgesehen, dass die L-Nukleinsäure ein Spiegelmer und die Verbindung das Zielmolekül des Spiegelmers ist.

Dabei ist in weiteren Ausführungsform vorgesehen, dass das Zielmolekül in einer biologischen Probe enthalten ist.

In einem noch weiteren Aspekt betrifft die vorliegenden Erfindung die Verwendung der erfindungsgemäßen Nukleinsäuren zur Herstellung eines Medikamentes.

In einem noch weiteren Aspekt betrifft die vorliegenden Erfindung die Verwendung der erfindungsgemäßen Nukleinsäuren zur Herstellung eines diagnostischen Mittels.

In einer bevorzugten Ausführungsform ist vorgesehen, dass die Nukleinsäure ein Spiegelmer ist, wobei das Spiegelmer gegen ein Zielmolekül gerichtet ist.

In einer weiteren Ausführungsform ist vorgesehen, dass das Zielmolekül an einem pathogenen Prozess beteiligt ist oder ein Marker für einen pathogenen Prozess darstellt.

Infolge der Zielmolekül-Spezifität der markierten L-Nukleinsäuren, insbesondere der solchermaßen markierten Spiegelmere, ist es möglich, den Ort des Zielmoleküls *in vivo,* genauer gesagt *in situ*, d. h. im lebenden Organismus, zu bestimmen durch bildgebende Verfahren, die hierin auch als in vivo imaging bezeichnet werden. Für diese bildgebenden Verfahren sind die prominentesten Beispiele die verschiedenen Resonanzverfahren wie magnetisches Resonanzverfahren sowie Positronenemissionstomographie. Insbesondere bei dem letzteren Verfahren werden Radionuklide benötigt, die beispielsweise mit den erfindungsgemäßen Verfahren in die L-Nukleinsäure eingeführt werden können. Neben dieser reinen darstellenden Funktion kann den solchermaßen markierten L-Nukleinsäuren jedoch auch noch eine Funktion dahingehend zukommen, dass diese bereits als therapeutisch wirksames Agens verwendet werden können. Grundsätzlich kann dies dadurch bedingt werden, dass anstelle eines für die bildgebenden Verfahren in der Regel vergleichsweise schwachen Strahlers ein stärkerer Strahler, oder eine andere Strahlerqualität, verwendet wird, um das Gewebe, an das die L-Nukleinsäure spezifisch bindet, durch radioaktive Strahlung zu behandeln.

Der vorliegenden Erfindung liegt die überraschende Erkenntnis zugrunde, dass es möglich ist, unter Verwendung einer T4-Polynukleotidkinase L-Nukleinsäuren zu markieren. Dabei erfolgt die Markierung typischerweise dadurch, dass ein eine Markierung tragendes Phosphat terminal an eine Nukleinsäure gebunden wird. Das Phosphat trägt, wie im folgenden ausgeführt ist, die eigentliche Markierung. Mittels dieses hierin auch als terminales Markierungsverfahren bezeichneten Verfahrens können somit L-Nukleinsäuren zuverlässig markiert werden, wobei der Akzeptor, d.h. der Teil, an den das Phosphat gebunden wird, selbst ein L-Nukleotid bzw. ein L-Nukleosid ist. Eine solchermaßen markierte L-Nukleinsäure trägt die Markierung terminal.

Der vorliegenden Erfindung liegt jedoch auch die Erkenntnis zugrunde, dass trotz der hohen Stabilität von L-Nukleinsäuren in biologischen Systemen eine Markierung an einem terminalen Phosphat der L-Nukleinsäure, insbesondere am 5'-terminalen Ende, wie sie gemäß der vorliegenden Erfindung erfolgen kann, und trotz der damit verbundenen großen Vorteile die Verwendung terminal markierter L-Nukleinsäuren mit gewissen Beschränkungen verbunden ist. Dies liegt in der überraschenden Feststellung begründet, dass die Esterbindung zwischen dem 5'-terminalen Phosphat und dem dazugehörigen L-Nukleosid durch unspezifische Phosphatasen gespalten werden kann, was zu einem Verlust der Markierung führt und somit die Anwendbarkeit markierter L-Nukleinsäuren wie Spiegelmere, insbesondere 5'-terminal markierter Spiegelmere, in biologischen Systemen insbesondere dann beschränken würde, wenn die solchermaßen markierten Spiegelmere über einen längeren Zeitraum zuverlässig mit der Markierung versehen sein müssten. Andererseits haben die Erfinder überraschenderweise festgestellt, dass diese unspezifische Spaltung des Phosphatesters innerhalb einer L-Nukleinsäure durch unspezifische Phosphatasen unterbleibt und somit eine stabile Markierung von L-Nukleinsäuren durch eine interne Markierung der L-Nukleinsäure möglich ist. Diese interne Markierung oder Markierung innerhalb von L-Nukleinsäuren, d.h. die nicht-terminale Markierung der L-Nukleinsäure kann durch das erfindungsgemäße Verfahren bewerkstelligt werden. Dabei haben die Erfinder wiederum überraschenderweise festgestellt, dass die T4-Polynukleotidkinase (E.C.-Nr. 2.7.1.78) auf 5'-Enden von L-Oligonukleotiden Phosphatreste übertragen kann, die beispielsweise von γ-[¹⁸O]-ATP, γ-[³²P]-ATP oder γ-[³³P]-ATP stammen. Darüber hinaus können neben dem Phosphat auch Thiophosphatreste, beispielsweise aus γ-[³⁵S]-ATP auf die L-Nukleinsäure übertragen werden. In all diesen Fällen stellt die Markierung im eigentlichen Sinne das radioaktive Nuklid ¹⁸O, ³²P, ³³P bzw. ³⁵S dar. Eine weitere Form der Markierung kann dadurch erfolgen, dass ATP-Derivate verwendet werden, die wiederum andere Isotope wie ¹³C, ¹⁴C, ³H, ¹⁵N oder ¹⁸O tragen, und diese sodann vermittels des übertragenen Phosphatrestes auch an die L-Nukleinsäure binden (Arzumanov AA, Semizarov DG, Victorova LS, Dyatkina NB, Krayevsky AA. Gamma-phosphate-substituted 2'-deoxynucleoside 5'-triphosphates as substrates for DNA polymerases. J Biol Chem. 1996; 271(40): 24389-94). Auch diese Art der Markierung kann sowohl intern in als auch terminal an einer L-Nukleinsäure erfolgen.

Infolge der Art der Markierung, wie sie hierin offenbart wird, dass nämlich die Struktur der L-Nukleinsäure durch die Markierung praktisch nicht verändert wird, wird auch gewährleistet, dass die L-Nukleinsäure hinsichtlich ihrer biologischen, chemischen und physikalischen Parameter nicht geändert wird und sich somit keinerlei Einschränkungen hinsichtlich der verschiedenen Anwendungen L-Nukleinsäure ergeben.

Die Erkenntnis, dass die T4-Polynukleotidkinase zur Markierung von L-Nukleinsäuren, insbesondere solchen, die ausschließlich aus L-Nukleotiden aufgebaut sind, verwendet werden kann, ist insoweit überraschend, als dass in Damha, M. J. et al., Tetrahedron Letters, vol. 32, no. 23, pp 2573-2576, 1991, offenbart wird, dass T4-Polynukleotidkinase ein L-Hexaoligonukleotid ausgehend von γ-³²P-ATP nicht phosphorylieren kann. Ebenso überraschend war für die Erfinder festzustellen, dass die Aussage von Williams K. P. et al., Proc. Natl. Acad. Sci. USA, vol. 94, pp 11285-11290, October 1997, Biochemistry, L-Nukleotide nicht zutreffend ist, dass die T4-Polynukleotidkinase zwei D-Thymidinreste am 5'-Ende eines Aptamers aus L-Nukleotiden benötigt, um das entsprechende L-Oligonukleotid als Substrat für die Übertragung eines Phosphat-Restes zu akzeptieren. Eine Übertragung eines Phosphatrestes auf ein Nukleosid eines Polynukleotides wird hierin auch als Kinasieren bezeichnet.

Nachdem Spiegelmere das Ergebnis eines Selektionsprozesses sind, bei dem eine Nukleinsäure durch Kontaktieren mit dem Zielmolekül aus einer Bibliothek von Nukleinsäuren erhalten wird, wäre die Einführung derartiger D-Thymidinreste am 5'-Ende lediglich zu Markierungszwecken, genauer gesagt um T4-Polynukleotidkinase zu Markierungszwecken verwenden zu können, nicht geeignet, um eine Markierung zu liefern, die genauso stabil ist, wie das Spiegelmer. Dies liegt darin begründet, dass die D-Thymidinreste ein Substrat für Nukleasen, wie sie typischerweise in biologischen Proben vorkommen, darstellen mit der Folge, dass bei der Verwendung solchermaßen markierter Nukleinsäuren wie Spiegelmeren, die Markierung schnell von der Nukleinsäure abgetrennt werden und es somit zu einer Entkopplung der Bindungseigenschaften der Nukleinsäure einerseits und dem Nachweis der Markierung andererseits kommen würde.

Bei den Zielmolekülen oder Targets kann es sich um Moleküle oder Strukturen handeln, wie z.B. um Viren, Viroide, Bakterien, Zelloberflächen, Zellorganellen, Proteine, Peptide, Nukleinsäuren, kleine Moleküle wie beispielsweise Metaboliten des Stoffwechsels, pharmazeutische Wirkstoffe oder deren Metabolite oder andere chemische, biochemische oder biologische Komponenten und Verbindungen.

Bei dem erfindungsgemäßen Verfahren zum internen Markieren von L-Nukleinsäuren wird die L-Nukleinsäure letzten Endes grundsätzlich aus zwei oder mehreren Teilen hergestellt, wobei die beiden Teile typischerweise innerhalb der insgesamt herzustellenden und eine Markierung aufweisenden Nukleinsäure nebeneinander angeordnet sind. Dabei ist wesentlich, dass zumindest ein Teil mit einem Marker versehen wird dergestalt, dass dieser Teil und damit die daran befestigte Markierung innerhalb der L-Nukleinsäure angeordnet ist. "Innerhalb der L-Nukleinsäure angeordnet" soll hierein insbesondere bedeuten, dass sich dem die Markierung tragenden Teil der L-Nukleinsäure wenigstens ein, bevorzugterweise mehrere Nukleotide anschließen und diese dann das Ende der Nukleinsäure darstellen oder ausbilden. Der Begriff "erster Teil" der zu markierenden L-Nukleinsäure bezeichnet somit hierin in erster Linie einen Teil einer L-Nukleinsäure, um diesen von weiteren Teilen der L-Nukleinsäure zu unterscheiden. Typischerweise handelt es sich bei dem ersten Teil um den 3' gelegenen Teil und bei dem zweiten Teil um den 5' gelegenen Teil der insgesamt herzustellenden und eine Markierung aufweisenden Nukleinsäure.

Die Länge der insgesamt herzustellenden und eine interne Markierung aufweisenden Nukleinsäure ist grundsätzlich nicht beschränkt und kann so wenig wie drei Nukleotide umfassen. Die Länge der Teile, aus denen die insgesamt herzustellende Nukleinsäure besteht oder hergestellt wird, ist prinzipiell nicht beschränkt, wobei jedoch bevorzugterweise der erste Teil eine Länge von etwa 7 bis etwa 70 Nukleotiden und der zweite Teil unabhängig davon ebenfalls eine Länge von etwa 7 bis etwa 70 Nukleotiden aufweist. Die Minimallänge eines jeden Teils der zu markierenden L-Nukleinsäure beträgt dabei 2 für den Fall, dass das erfindungsgemäße Verfahren zur internen Markierung verwendet wird. Es ist jedoch in Anwendung des erfindungsgemäßen terminalen Markierungsverfahrens auch möglich, dass ausgehend von einem L-Dinukleotid eine Markierung auf dieses übertragen wird und sodann das Dinukleotid mit einem weiteren L-Nukleotid umgesetzt wird. In der Folge wird ein intern markiertes Trinukleotid erhalten werden.

Der Begriff "L-Nukleinsäure" bezeichnet hierin sowohl L-Desoxyribonukleinsäure wie auch L-Ribonukleinsäure und Kombinationen davon, d. h. dass einzelne oder eine Gruppe von Nukleotiden als RNA vorliegen, und die weiteren die Nukleinsäure aufbauenden Nukleotide als DNA vorliegen und umgekehrt. Es ist im Rahmen der vorliegenden Erfindung, dass die Markierung nicht-terminal angebracht ist, d. h. innerhalb einer Abfolge von L-Nukleotiden, jedoch kann die insgesamt markierte L-Nukleinsäure darüber hinaus sehr wohl Bereiche umfassen, worin statt L-Nukleotiden D-Nukleotide enthalten sind.

Die L-Nukleinsäure kann grundsätzlich doppel- oder einzelsträngig vorliegen. Typischerweise handelt es sich um eine einzelsträngige L-Nukleinsäure, die jedoch bedingt durch ihre Primärsequenz definierte Sekundärstrukturen und damit auch Tertiärstrukturen ausbilden kann. In der Sekundärstruktur liegen bei einer Vielzahl von L-Nukleinsäuren auch doppelsträngige Abschnitte vor.

Die L-Nukleinsäuren können neben der Markierung eine Modifizierung aufweisen. Eine derartige Modifikation kann sich dabei auf die einzelnen Nukleotide der Nukleinsäure beziehen und sind in der Technik gut bekannt. Beispiele für derartige Modifikationen finden sich in Kusser, W.(2000) J Biotechnol, 74: 27-38; Aurup, H. et al. **(1994)** *Nucleic Acids Res,* **22,** 20-4; Cummins, L.L. et al, **(1995)** *Nucleic Acids Res,* **23,** 2019-24; Eaton, B.E. et al. **(1995)** *Chem Biol,* **2,** 633-8; Green, L.S. et al., **(1995)** *Chem Biol,* ***2,*** 683-95; Kawasaki, A.M. et al., **(1993)** *J Med Chem,* 36, 831-41; Lesnik, E.A. et al., **(1993)** *Biochemistry,* **32,** 7832-8; Miller, L.E. et al., **(1993)** *J Physiol,* **469,** 213-43.

Handelt es sich bei der zu markierenden L-Nucleinsäure um eine L-RNA, wird bevorzugterweise der chemische Ligationsort derart gewählt, dass auch eine 2'-5'-Verknüpfung die Bindungs- oder enzymatischen Eigenschaften des Moleküls nicht gravierend verändert.

Weiterhin kann vorgesehen sein, dass der zu ligierende Strang, dessen Ligationsstelle am 3'-Ende liegt, eine 2'-Modifizierung aufweist, die eine Schutzgruppe sein kann, also nach der Reaktion wieder abgespalten werden kann, oder eine permanente Modifizierung darstellt, die aus der Gruppe -H (desoxy-), -OMe, - N₃, oder anderen bekannten 2'-Modifikationen stammt, die vorzugsweise die Eigenschaften des Moleküls nicht bzw. nur geringfügig verändern.

Nach durchgeführter chemischer Ligation kann die entsprechende Matrix nicht-hydrolytisch gespalten werden, so daß deren Abtrennung durch Denaturierung und anschließende Reinigung erfolgt.

Insbesondere bei einer radioaktiven Markierung der L-Nukleinsäure unter Verwendung einer enzymatischen Aktivität, wobei hier wiederum besonders bevorzugt die T4-Polynukleotid-Kinase ist, ist es infolge der Spezifität der von der Kinase katalysierten Reaktion erforderlich, dass eine Markierung des ersten Teiles der zu markierenden L-Nukleinsäure am 5'-Ende erfolgt, typischerweise in Form eines radioaktiv markierten Phosphates oder Thiophosphates. Für die Markierung mittels der Kinase ist es jedoch auch möglich, einen geeigneten anderen Donor für die Markierung zu verwenden, solange dieser Donor von der Kinase als Substrat akzeptiert wird. Typischerweise handelt es sich bei den Substraten um γ-[¹⁸O]-ATP, γ-[³²P]-ATP, γ-[³³P]-ATP und γ-[³⁵S]-ATP. Die Markierungen im eigentlichen Sinne stellen dabei die entsprechenden Nuklide dar, d. h. ¹⁸O, ³²P, ³³P und ³⁵S. Weitere Substrate können Derivate von ATP darstellen, die andere oder weitere Radionuklide als die vorstehend erwähnten Radionuklide tragen. Derartige Radionuklide können ¹³C, ¹⁴C, ¹⁵N oder ³H sein, die dadurch in die zu markierende L-Nukleinsäure eingeführt werden können, dass sie in oder an dem typischerweise durch die Kinase übertragenen terminalen Phosphat vorhanden sind. Derartige Derivate des ATP bzw. des Phosphates sind gamma-Phosphonate. Es ist für den Fachmann offensichtlich, dass das gleichzeitige Vorhandensein zweier oder mehrerer Radionuklide innerhalb einer Nukleinsäure mit Vorteilen verbunden ist.

Die Einführung der radioaktiven Markierung kann auch durch eine chemische Reaktion, also eine nicht durch ein Enzym vermittelte Reaktion zustandekommen (Z.Shabarova, A.Bogdanov, Advanced Organic Chemistry of Nucleic Acids, VCH Weinheim, 1994, 1. Aufl.), zum Beispiel durch die Reaktion des 5'-Hydroxyls der L-Nukleinsäure mit einem Carbodiimid oder einem anderen Kopplungsreagenz und anorganischem markierten Phosphat, oder durch Umsetzen eines aktivierten markierten Phosphatderivates wie einem Phosphorsäureimidazolides mit der L-Nukleinsäure.

Die terminale Phosphatgruppe kann auch durch radioaktive Verbindungen derivatisiert werden, zum Beispiel durch die Interaktion von markiertem CH₃OH (oder anderen Alkoholen, Aminen, Thiolen) und einem Carbodiimid, oder der Umsetzung eines aktivierten 5'-Phosphates mit radioaktiv markierten Alkoholen, Aminen oder Thiolen.

Bei den chemischen Modifizierungen kann vorgesehen sein, dass diese durchgeführt wird, wenn die entsprechende L-Nukleinsäure noch ihre Schutzgruppen nach der Festphasensynthese trägt.

Die Reaktion der T4-Polynukleotid-Kinase, wie sie beispielsweise für den Transfer eines terminalen Phosphats auf ein D-Oligonukleotid beschrieben ist in Sambrook, Fritsch, Maniatis, Molecular Cloning- A laboratory Manual, 2^{nd} Ed. 1989, Cold Spring Harbor Laboratory Press, p.5.68), erfolgt unter Standardbedingungen auch für den Transfer des γ-Phosphats eines Nukleosidtriphosphates auf die 5'-Hydroxylgruppe der L-Nukleinsäure. Wie in den Beispielen weiter ausgeführt werden wird, weist die T4-Polynukleotid-Kinase eine Präferenz für verschiedene terminale Nukleotide auf. So wird 5'-terminal gelegenes Cytosin und Guanosin gegenüber Adenosin und Thymidin bevorzugt, wobei jedoch auch diese Nukleotide grundsätzlich verwendet werden können.

Infolge dieser Präferenz der T4-Polynukleotidkinase für 5'-terminale Cytosinresten bzw. Guanosinresten ist es bevorzugt, eine mit einer Markierung zu versehende L-Nukleinsäure in mindestens zwei Teile oder Teilsequenzen aufzuteilen, wobei das 5'-Ende des einen Teils, das später innerhalb der markierten vollständigen L-Nukleinsäure vorliegen wird, entweder einen Guanosin- oder einen Cytosinrest trägt.

Die Herstellung der zu markierenden L-Nukleinsäure ist in der Technik bekannt und beispielsweise beschrieben in WO 98/08856. Entsprechend wird bei der Markierung von L-Nukleinsäuren ein erster Teil und ein zweiter Teil der insgesamt zu synthetisierenden und die Markierung letzten Endes aufweisenden L-Nukleinsäure synthetisiert, wobei der aneinandergefügte erste Teil und zweite Teil die insgesamt herzustellende L-Nukleinsäure darstellt bzw. ausbildet. Es ist auch im Rahmen der vorliegenden Erfindung, dass an diese L-Nukleinsäuresequenz weitere Nukleotide oder Oligonukleotide hinzugefügt werden, die ihrerseits eine terminale oder eine interne Markierung aufweisen können. Durch diese Maßnahme können weitere Markierungen in die Nukleinsäure eingebracht werden, die untereinander entweder gleich oder verscheiden sein können. Durch die weitere Einführung von Markierungen kann beispielsweise die Signalintensität und das Signal-Rausch-Verhältnis erhöht werden.

Die Ligation, insbesondere die chemische Ligation von zwei Oligonukleotiden, wie sie bei dem erfindungsgemäßen Verfahren als ein Teilschritt vorgesehen ist, ist in der Literatur als solches bekannt (Dolinnaya, N. G., Sokolova, N. I., Ashirbekova, D. T., Shabarova, Z. A. (1991) The use of BrCN for assembling modified DNA duplexes and DNA-RNA hybrids; comparison with water-soluble carbodiimide. *Nucleic Acids Res.,* **19**, 3067-3072) und kann auch auf L-Nukleinsäuren angewandt werden. Neben der Verwendung von BrCN, Mischungen aus BrCN, Ni²⁺ ( oder anderen divalenten Metallionen, wie Co²⁺, Mn²⁺, Mg²⁺, Cu²⁺, Zn²⁺) und Imidazol oder Imidazolderivaten ( wie z.B. N-Cyanoimidazol, N,N'-Iminodiimidazol) (E. Kanaya, H. Yanagawa, Template-directed polymerization of oligoadenylates using cyanogen bromide, Biochemistry (1986), 25, 7423-7430) bzw., sind weitere Kopplungsreagenzien in der Technik bekannt, so beispielsweise Carbodiimide , bevorzugt wasserlösliche Carbodiimide ( wie EDC und CMC- N-Cyclohexyl-N'morpholinoethyl-carbodiimid).

Im Rahmen des Ligationsschrittes kann im Rahmen des erfindungsgemäßen Verfahrens auf die sog. Matrix-gestützte chemische Ligation zurückgegriffen werden, die im Stand der Technik als solche bekannt ist (Dolinnaya, N. G., Sokolova, N. I., Ashirbekova, D. T., Shabarova, Z. A. (1991) The use of BrCN for assembling modified DNA duplexes and DNA-RNA hybrids; comparison with water-soluble carbodiimide (*Nucleic Acids Res.,* **19**, 3067-3072). Dabei wird eine Matrix bereitgestellt, die zumindest teilweise komplementär ist zu den mindestens beiden zu ligierenden Teilen der insgesamt herzustellenden und zu markierenden L-Nukleinsäure. Dabei ist es ausreichend, wenn diese Komplementarität zu einem jeden der beiden Teile lediglich teilweise gegeben ist. Der genaue Umfang der Überlappung hängt von den jeweiligen Reaktionsbedingungen ab und kann vom Fachmann leicht durch Routineversuche bestimmt werden. Typischerweise wird man von einer Überlappung auf einer jeden Seite von etwa vier Nukleotiden ausgehen.

Um nach erfolgreicher Ligation die Dissoziation des Produkt-Matrix-Duplex zu fördern, kann vorgesehen sein, dass die Matrix, sofern diese als Desoxyribonukleinsäure ausgebildet ist, zumindest ein Ribonukleosid enthält, wie beispielsweise Riboadenosin, welches anschließend gespalten wird, um eine Auftrennung mittels geeigneter Trennverfahren, wie beispielsweise Polyacrylamidgelelektrophorese (PAGE), HPLC oder FPLC), zu ermöglichen.

Bei den hierin beschriebenen markierten L-Nukleinsäuren handelt es sich bevorzugterweise um sog. Spiegelmere. Wie eingangs bereits erwähnt, sind Spiegelmere funktionale Nukleinsäuren, d. h. solche, die an ein Zielmolekül oder einen Teil davon binden, und das Ergebnis des Kontaktierens einer Nukleinsäurebibliothek, insbesondere einer statistischen Nukleinsäurebibliothek, mit dem Zielmolekül sind.

Für ein Selektionsverfahren zur Entwicklung funktionaler Nukleinsäuren werden zunächst kombinatorische DNA-Bibliotheken hergestellt. In der Regel handelt es sich um die Synthese von DNA-Oligonukleotiden, die zentral einen Bereich aus 10 -100 randomisierten Nukleotiden enthalten, die von zwei primer-Bindungsregionen 5'- und 3'-terminal flankiert werden. Die Herstellung derartiger kombinatorischer Bibliotheken ist bspw. beschrieben in Conrad, R.C., Giver, L., Tian, Y. and Ellington, A.D., 1996, Methods Enzymol., Vol 267, 336-367. Eine solche chemisch synthetisierte einzelsträngige DNA-Bibliothek lässt sich über die Polymerase-Kettenreaktion in eine doppelsträngige Bibliothek überführen, die für sich genommen schon für eine Selektion eingesetzt werden kann. In der Regel erfolgt jedoch mit geeigneten Methoden eine Separation der einzelnen Stränge, so dass man wieder zu einer Einzelstrangbibliothek gelangt, die für das in vitro Selektionsverfahren eingesetzt wird, wenn es sich um eine DNA-Selektion handelt (Bock, L.C., Griffin, L.C., Latham, J.A., Vermaas, E.H. und Toole, J.J., 1992, Nature, Vol. 355, 564-566). Es ist jedoch ebenso möglich, die chemisch synthetisierte DNA-Bibliothek direkt in die in vitro Selektion einzusetzen. Darüber hinaus kann prinzipiell aus doppelsträngiger DNA, wenn zuvor ein T7 Promotor eingeführt worden ist, auch über eine geeignete DNA-abhängige Polymerase, z. B. die T7 RNA Polymerase, eine RNA-Bibliothek erzeugt werden. Mit Hilfe der beschriebenen Verfahren ist es möglich, Bibliotheken von 10¹⁵ und mehr DNA- oder RNA-Molekülen zu erzeugen. Jedes Molekül aus dieser Bibliothek hat eine andere Sequenz und somit eine andere dreidimensionale Struktur. Über das in vitro Selektionsverfahren ist es nun möglich, aus der erwähnten Bibliothek durch mehrere Zyklen von Selektion und Amplifikation sowie gegebenenfalls Mutation ein oder mehrere DNA-Moleküle zu isolieren, die gegen ein gegebenes Target eine signifikante Bindungseigenschaft aufweisen. Die Targets können z. B. Viren, Proteine, Peptide, Nukleinsäuren, kleine Moleküle wie Metaboliten des Stoffwechsels, pharmazeutische Wirkstoffe oder deren Metaboliten oder andere chemische, biochemische oder biologische Komponenten sein wie beispielsweise in Gold, L., Polisky, B., Uhlenbeck, O. und Yarus, 1995, Annu. Rev. Biochem. Vol. 64, 763-797 und Lorsch, J.R. und Szostak, J.W., 1996, Combinatorial Libraries, Synthesis, Screening and application potential, ed. Riccardo Cortese, Walter de Gruyter, Berlin, beschrieben. Das Verfahren wird in der Weise durchgeführt, dass bindende DNA- oder RNA-Moleküle aus der ursprünglich eingesetzten Bibliothek isoliert werden und nach dem Selektionsschritt mittels Polymerase-Kettenreaktion amplifiziert werden. Bei RNA-Selektionen ist vor dem Amplifikationsschritt durch Polymerase-Kettenreaktion eine reverse Transkription vorzuschalten. Eine nach einer ersten Selektionsrunde angereicherte Bibliothek kann dann in eine erneute Selektionsrunde eingesetzt werden, so dass die in der ersten Selektionsrunde angereicherten Moleküle die Chance haben, durch Selektion und Amplifikation sich erneut durchzusetzen und mit noch mehr Tochtermolekülen in eine weitere Selektionsrunde zu gehen. Gleichzeitig eröffnet der Schritt der Polymerase-Kettenreaktion die Möglichkeit, neue Mutationen bei der Amplifikation z.B. durch die Variation der Salzkonzentration einzubringen. Nach genügend vielen Selektions- und Amplifikationsrunden haben sich die bindenden Moleküle durchgesetzt. Es ist so ein angereicherter Pool entstanden, dessen Vertreter durch Klonierung vereinzelt und anschließend mit den gängigen Methoden der Sequenzbestimmung von DNA in ihrer Primärstruktur bestimmt werden können. Die erhaltenen Sequenzen werden dann auf ihre Bindungseigenschaften hinsichtlich des Targets überprüft. Das Verfahren zur Erzeugung derartiger Aptamere wird auch als SELEX-Verfahren bezeichnet und ist bspw. beschrieben in EP 0 533 838, dessen Offenbarung hierin durch Bezugnahme aufgenommen wird.

Die besten Bindungsmoleküle können durch Verkürzung der Primärsequenzen auf ihre wesentliche Bindungsdomäne hin verkürzt und durch chemische oder enzymatische Synthese dargestellt werden.

Eine besondere Form von solchermaßen herstellbaren Aptameren sind die sogenannten Spiegelmere, die sich im wesentlichen dadurch auszeichnen, dass sie zumindest teilweise, bevorzugt vollständig aus den nicht-natürlichen L-Nukleotiden aufgebaut sind. Verfahren zur Herstellung derartiger Spiegelmere sind beschrieben in PCT/EP97/04726, deren Offenbarung hiermit durch Bezugnahme aufgenommen wird. Die Besonderheit des darin beschriebenen Verfahrens liegt in der Erzeugung von enantiomeren Nukleinsäuremolekülen, d.h. von L-Nukleinsäuremolekülen, die an ein natives, d.h. in der natürlichen Form oder Konfiguration vorliegendes Target oder eine derartige Targetstruktur binden. Das oben beschriebene in vitro Selektionsverfahren wird dazu eingesetzt, bindende Nukleinsäuren oder Sequenzen zunächst gegen die enantiomere, d.h. nicht natürlich vorkommenden Struktur eines natürlicherweise vorkommenden Targets zu selektieren, beispielsweise im Falle, dass das Zielmolekül ein Protein ist, gegen ein D-Protein. Die so erhaltenen Bindungsmoleküle (D-DNA, D-RNA bzw. entsprechende D-Derivate) werden in ihrer Sequenz bestimmt und die identische Sequenz wird dann mit spiegelbildlichen Nukleotidbausteinen (L-Nukleotide bzw. L-Nukleotidderivate) synthetisiert. Die so erhaltenen spiegelbildlichen, enantiomeren Nukleinsäuren (L-DNA, L-RNA bzw. entsprechende L-Derivate), sogenannte Spiegelmere, haben aus Symmetriegründen eine spiegelbildliche Tertiärstruktur und somit eine Bindungseigenschaft für das in der natürlichen Form oder Konfiguration vorliegende Target.

Der Begriff L-Nukleinsäuren wird hierin im Sinne von L-Polynukleotiden verwendet und umfasst insbesondere auch solche Verbindungen, bei denen der Zuckerrest eines oder mehrerer Nukleotide verschieden ist von Ribose oder Deoxyribose.

Die Erfindung wird nun anhand der folgenden Figuren und Beispiele weiter erläutert, aus denen sich weitere Merkmale, bevorzugte Ausführungsformen und Vorteile der Erfindung ergeben. Dabei zeigt
- Fig. 1a: ein Schema zur Veranschaulichung des erfindungsgemäßen Verfahrens zur Markierung von Deoxyribo-L-Nukleinsäuren,
- Fig. 1b: ein Schema zur Veranschaulichung des erfindungsgemäßen Verfahrens zur Markierung von L-Ribonukleinsäuren,
- Fig. 2: ein Autoradiogramm einer 20 % PAGE nach einer Kinasierung von DNA-Spiegelmeren gemäß SEQ ID No.1 - 4,
- Fig. 3: ein Autoradiogramm einer 10 % PAGE nach einer Kinasierung von DNA-Spiegelmeren gemäß SEQ ID No.6,
- Fig. 4: ein Autoradiogramm einer 10 % PAGE nach einer chemischen Ligation der DNA-Spiegelmere gemäß SEQ ID No.5 und 6 mittels einer Matrix mit einer Nukleinsäuresequenz gemäß SEQ ID No.7, und
- Fig. 5: eine Darstellung der Ligation unter Verwendung der Nukleinsäuresequenz gemäß SEQ ID No.7 als komplementäre Matrix und Ligation der Nukleinsäure entsprechend den Nukleinsäuresequenzen SEQ ID No.5 und 6,
- Fig. 6a: eine schematische Darstellung des erfindungsgemäßen Verfahrens zur internen Markierung von L-Desoxyribonukleinsäuren, wobei mehrere interne Markierungen in eine L-Desoxyribonukleinsäure eingeführt werden und
- Fig. 6b: eine schematische Darstellung des erfindungsgemäßen Verfahrens zur internen Markierung von L-Ribonukleinsäuren, wobei mehrere interne Markierungen in eine L-Ribonukleinsäure eingeführt werden und
- Fig. 7: eine schematische Darstellung der terminalen Markierung einer L-Nukleinsäure mit längerlebigen Isotopen und die Synthese der entsprechenden Bausteine.

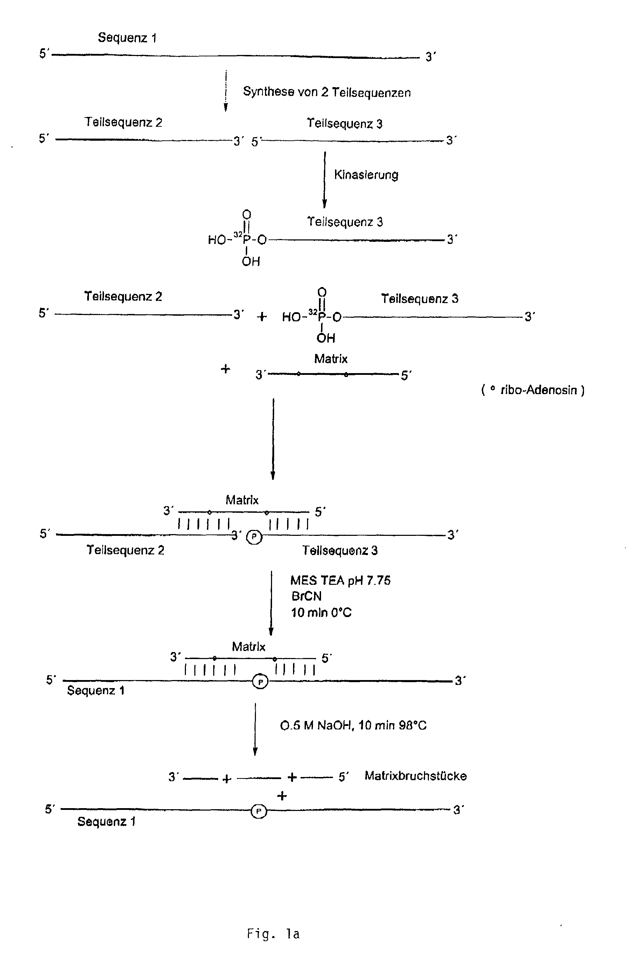

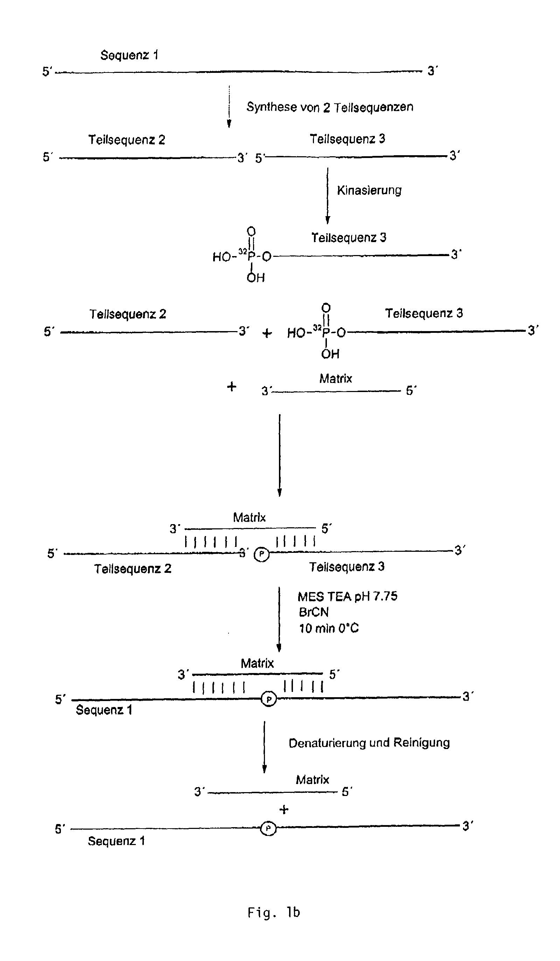

Das erfindungsgemäße Verfahren ist schematisch in den Figs. 1a und 1b anhand der Markierung eines Spiegelmers dargestellt, wobei das Verfahren grundsätzlich auf eine jegliche L-Nukleinsäure anwendbar ist. Ausgehend von einem Spiegelmer, welches eine Sequenz 1 aufweist und typischerweise im Rahmen eines Selektionsverfahrens erhalten wurde, soll eine Markierung in das Spiegelmer eingeführt werden bzw. ein derartiges, mit einer Markierung versehenes Spiegelmer hergestellt werden. Dazu wird die Sequenz in zwei Teilsequenzen 2 und 3 aufgetrennt. Die beiden Teilsequenzen 2 und 3 weisen eine Länge von mehreren Nukleotiden auf. Diese Aufteilung oder Zergliederung der Nukleinsäure in zwei (oder mehrere Teile) erfolgt typischerweise lediglich theoretisch. Die Aufteilung orientiert sich dabei an der Sequenz der zu markierenden Nukleinsäure, wobei die Präferenzen der verwendeten enzymatischen Aktivität wie beispielsweise der T4-Polynukleotidkinase, berücksichtigt werden. Besonders bevorzugte Stellen innerhalb der Nukleinsäuresequenz sind dabei Stellen mit Guanosin oder Cytosin, besonders bevorzugt sind Stellen mit zwei aufeinanderfolgenden Guanosin-Resten.

Die Teilsequenz 3, welche hierin auch als erster Teil der zu markierenden L-Nukleinsäure bezeichnet wird, wird sodann markiert, im vorliegenden Fall mit der T4-Polynukleotidkinase unter Verwendung von γ-³²P-ATP als Phosphat-Donor. Nach der enzymatischen Umsetzung trägt die Teilsequenz 3 eine radioaktive Markierung in Form des in dem 5'-terminalen Phosphatrestes enthaltenen ³²P. Die solchermaßen kinasierte Teilsequenz 3 wird anschließend mit Teilsequenz 2 ligiert. Dies erfolgt unter Verwendung einer komplementären Matrix 4, die zumindest teilweise komplementär zu mindestens einem Teil der Teilsequenz 2 und/oder der Teilsequenz 3 ist. Beide Teilsequenzen 2 und 3 hybridisieren mit der Matrix 4 und werden somit in räumlicher Nähe zueinander angeordnet. In Folge dieser räumlichen Nähe kann eine chemische Ligation erfolgen. Im vorliegenden Fall wird als Kopplungsreagenz Bromcyan (10 min, 0°C) eingesetzt.

Nach der Ligation von Teilsequenz 2 und 3 liegt die zu markierende L-Nukleinsäure 1 auch physikalisch vor, die jedoch noch an die Matrix 4 gebunden ist. Die Abtrennung von Matrix 4 kann beispielsweise durch Denaturierung (95-98°C, 4M Hamstofflösung) infolge Erhöhung der Temperatur erfolgen. Alternativ oder in Ergänzung dazu kann die Matrix selbst gespalten werden. Dies erfolgt dadurch, dass für den Fall, dass die Matrix aus L-DNA besteht zumindest ein Nukleotid als Ribonukleotid ausgebildet ist und somit eine erhöhte Hydrolysierbarkeit unter basischen Bedingungen gegeben ist. Infolge dieser Hydrolyse kommt es zu einer Verringerung der Länge der die Matrix aufbauenden Nukleinsäure mit der Folge, dass eine leichtere Abtrennung der markierten Nukleinsäure 1 und damit Aufreinigung der markierten Nukleinsäure 1 beispielsweise durch Polyacrylamidgelelektrophorese möglich wird.

Hinsichtlich der Kombinationsmöglichkeiten zwischen der Art der Matrix, d. h. Ausbildung derselben als (L-)Desoxyribonukleinsäure oder (L-)Ribonukleinsäure, bestehen grundsätzlich keine Beschränkungen, so dass die folgenden vier Möglichkeiten gegeben sind: Matrix: Ribonukleinsäure, zu markierende bzw. markierte L-Nukleinsäure: L-Ribonukleinsäure oder L-Deoxyribonukleinsäure; Matrix: Deoxyribonukleinsäure und markierte bzw. zu markierende L-Nukleinsäure, L-Deoxyribonukleinsäure oder L-Ribonukleinsäure.

Das in Fig. 1b dargestellte Verfahren unterscheidet sich von dem in Fig. 1a dargestellten Verfahren nur darin, dass eine L-Ribonukleinsäure statt einer L-Deoxyribonukleinsäure modifiziert werden soll. Nachdem L-Ribonukleinsäuren unter basischen Bedingungen gespalten werden können, erfolgt die Abtrennung der Matrix von der ligierten und markierten Sequenz 1 im Rahmen des in Fig. 1b dargestellten Verfahrens durch Denaturierung. Die Matrix kann in diesem Falle entweder aus Ribonukleotiden oder Deoxyribonukleotiden oder beiden aufgebaut sein.

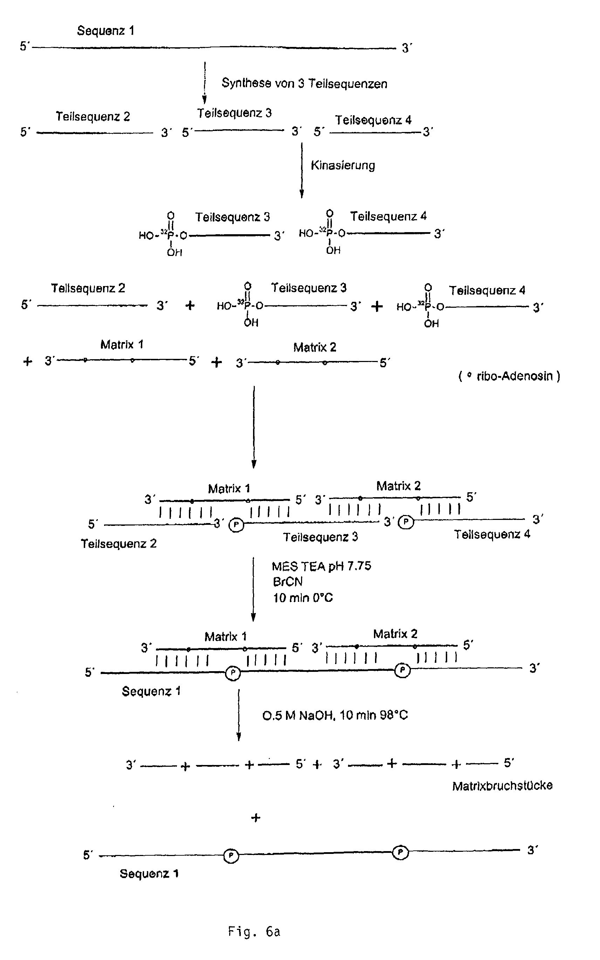

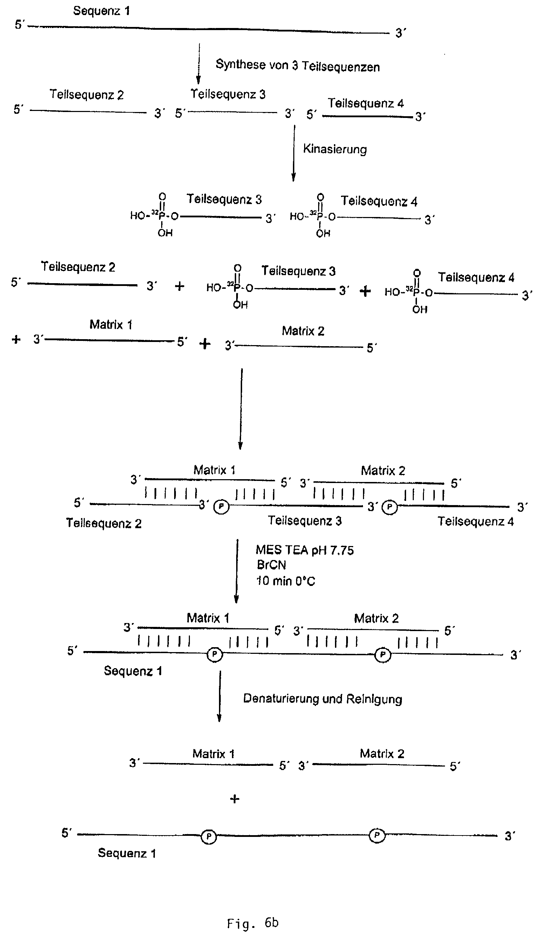

Bei den in den Figs. 6a und 6b gezeigten schematischen Darstellung des erfindungsgemäßen Verfahrens zur internen Markierung von L-Nukleinsäuren handelt es sich um eine weitere Ausführungsform, bei der insgesamt mehrere interne Markierungen in eine L-Nukleinsäure eingeführt werden. Die insgesamt herzustellende und intern zu markierende Nukleinsäure, in den Figs. 6a und 6b als Sequenz 1 bezeichnet, wird aus insgesamt drei Teilstücken (Teilsequenz 2, Teilsequenz 3 und Teilsequenz 4) zusammengesetzt. Die Teilsequenzen 3 und 4 werden gemäß dem erfindungsgemäßen terminalen Markierungsverfahren, welches einen Teilschritt des erfindungsgemäßen Verfahrens zum internen Markieren von L-Nukleinsäuren darstellt, am 5'-Ende mit einem Phosphatrest vermittels einer Kinase versehen. Die eigentliche Markierung stellt das Radionuklid des Phosphors (³²p) dar.

In einem weiteren Schritt werden sodann die Matrix 1 und 2 bereitgestellt, wobei die Matrix 1 sowohl eine zu mindestens einem Teil der Teilsequenz 2 zumindest teilweise komplementäre wie auch zu mindestens einem Teil der Teilsequenz 3 zumindest teilweise komplementäre Sequenz aufweist. Weiterhin weist die Matrix 1 im Falle des in Fig. 6a dargestellten Verfahrens mehrere ribo-Adenosin-Reste auf, die, wie bereits im Zusammenhang mit den Figs. 1a und 1b ausgeführt, zu einer Hydrolyse der Matrix und damit leichteren Abtrennbarkeit des Ligationsproduktes aus den Teilsequenzen von der Matrix führt. Die Matrix 2 weist einen zur Matrix 1 grundsätzlich ähnlichen Aufbau auf, wobei die Matrix 2 sowohl eine zu mindestens einem Teil der Teilsequenz 3 zumindest teilweise komplementäre wie auch zu mindestens einem Teil der Teilsequenz 4 zumindest teilweise komplementäre Sequenz aufweist. Durch Hybridisierung der Matrix mit den jeweiligen Teilsequenzen kann die Ligation durchgeführt werden. Dabei ist es durchaus möglich, dass zuerst eine Ligation zwischen den Teilsequenzen 2 und 3 und anschließend mit Teilsequenz 4 erfolgt oder eine Ligation zwischen den Teilsequenzen 3 und 4 und anschließend mit Teilsequenz 2. Die weiteren Reaktionsschritte entsprechen grundsätzlich den im Rahmen der von Figs. 1a und 1b beschriebenen.

Die in den Figs. 6a und 6b dargestellten Verfahren unterscheiden sich insbesondere hinsichtlich der Abtrennung der Matrix von der markierten Nukleinsäure Sequenz 1. Im Rahmen des in Fig. 6a dargestellten Verfahrens, bei dem eine Deoxynukleinsäure markiert wird, enthält sowohl Matrix 1 als auch Matrix 2 mindestens ein Ribonukleotid. Diese erlauben, dass die Matrix zur Abtrennung derselben von der markierten Sequenz 1 unter den angegebenen Bedingungen hydrolysiert wird. Die Abtrennung der Matrix von der markierten Sequenz 1 im Rahmen des in Fig. 6b dargestellten Verfahren zur Markierung einer L-Ribonukleinsäure erfolgt hingegen unter denaturierenden Bedingungen, da eine Hydrolyse der Matrix, wie sie bei der Ausgestaltung der Matrix wie im Falle des in Fig. 6a dargestellten Verfahrens möglich wäre, bei der Markierung von L-Ribonukleinsäure nicht in Frage kommt, da ansonsten auch die markierte L-Nukleinsäure wieder hydrolysiert würde.

Hinsichtlich der verschiedenen Kombinationsmöglichkeiten von Matrix und zu markierender Nukleinsäure gelten die zu den in den Figs. 1a und 1b dargestellten Verfahren gemachten Ausführungen entsprechend

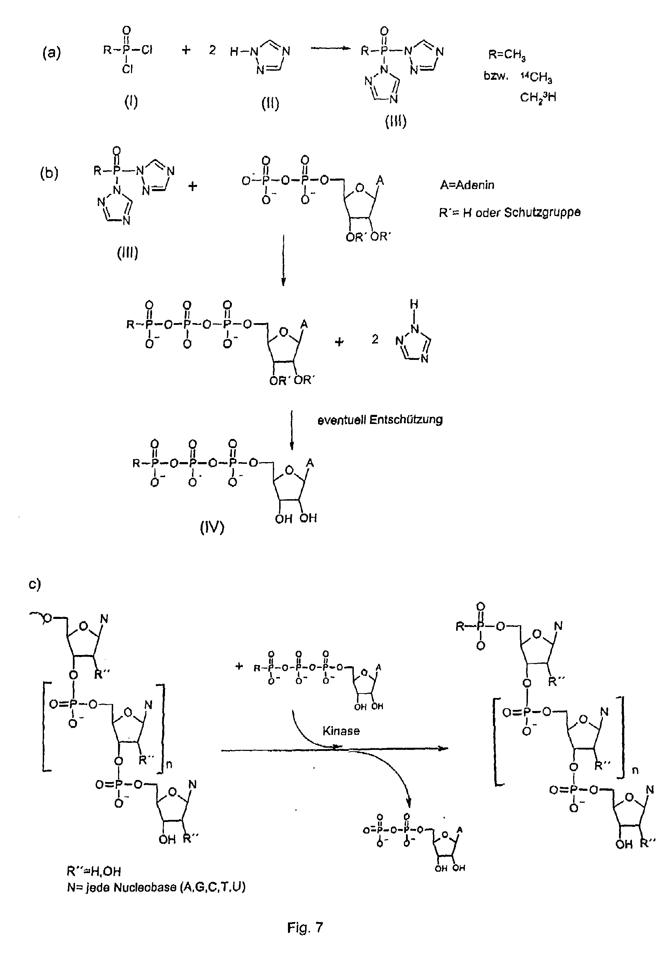

Fig. 7 beschreibt eine schematische Darstellung der erfindungsgemäßen terminalen Markierung einer L-Nukleinsäure. In Teilschritt (a) wird ausgehend von Methylphosphoroxydichlorid (I) und 1,2,4-Triazol (II) ein Phosphonsäure-bis-triazolid (III) hergestellt. Die Reaktionsbedingungen hierfür sind dem Fachmann bekannt. Die Methylgruppe von (I) enthält dabei das Radionuklid, beispielsweise ¹³C, ¹⁴C oder ³H. Es ist auch möglich, dass die Methylgruppe mehr als ein Radionuklid trägt. Weiterhin ist es möglich, dass auch der Phosphor selbst ein Radionuklid wie ³²P oder ³³P ist. Es ist anzumerken, dass die hierin offenbarten Phosphonat-Derivate neben einer Methylgruppe sowie deren Derivaten auch andere Gruppen umfassen kann, beispielsweise Ethyl-, Propyl-, Isopropyl-, Butanyl-Gruppen, die ihrerseits wiederum derivatisiert sein können. Weitere Möglichkeiten der Derivatisierung der Phosphonsäure mit dem Ziel, Radionuklide in das terminale Phosphat eines Adenosintriphosphates einzuschleusen wiederum mit dem Ziel, durch Übertragung des γ-Phosphonates ein Radionuklid auf die Akzeptor-Verbindung zu übertragen, sind den Fachleuten auf dem Gebiet bekannt. In einem weiteren Schritt (b) wird die Verbindung (III) mit Adenosindiphosphat umgesetzt. Die beiden OH-Gruppen in 2' und 3' -Position des Riboseteils können dabei mit den aus der Nukleotidsynthese bekannten Schutzgruppen versehen sein. Das Reaktionsprodukt ist ein Adenosintriphosphat-Derivat, genauer gesagt ein Adenosinphosphonat (IV).

Das Adenosinphosphonat wird in einem Schritt (c) mit einer zu markierenden Nukleinsäure sowie einer Kinase umgesetzt, wobei das 5'-terminale Nukleotid der Nukleinsäure eine freie 5'-OH-Gruppe an seinem Zuckerrest aufweist, wobei der Zucker sowohl eine Ribose als auch eine Desoxyribose sein kann. Infolge der Kinase-Reaktion wird das γ-Phosphonat des Adenosinphosphonats (IV) auf die 5'-OH-Gruppe übertragen und somit die Nukleinsäure markiert. Es ist für den Fachmann offensichtlich, dass die Reaktionsbedingungen und Ausgestaltungen dieser Markierungsreaktion für diese Art der terminalen Markierung genauso ausgestaltet werden können, wie sie im Zusammenhang mit dem erfindungsgemäßen Verfahren zur internen Markierung von L-Nukleinsäuren hierin beschrieben sind.

### Beispiel 1: Kinasierung von L-Oligonukleotiden

Jegliche L-Oligonukleotide wurden auf einem ABI 394 DNA Synthesizer in einem 0.2 oder 1.0 µmol Maßstab unter Verwendung der Standard-Phosphoramiditchemie hergestellt. L-DNA-Phosphoramidite wurden von ChemGenes Corporation bezogen.

Die folgenden L-Nukleotidsequenzen wurden hergestellt:

1 5'-GGATT ACA GCG CTG-3'(SEQ. ID No.1)
2 5'-CGA GTT ACA GCG CTG-3' (SEQ. ID No.2)
3 5' -AGA GTT ACA GCG CTG-3' (SEQ. ID No.3)
4 5'-TGA GTT ACA GCG CTG-3'(SEQ. ID No.4)
5 5'-CCA AGC TTG CGT AAG CAG TCT CCT CTC AGG-3'(SEQ. ID No.5)
6 5'-GGA GGT TGG GCG GTG CGT AAG CAC CGG TTT GCA GGG G -3'(SEQ. ID
No.6)
7 5'-TTA CGC (rA)CC GCC CA(rA) CCT CCC CTG (rA)GA GGA GAC-3' (SEQ. ID No.7)

Die Oligonukleotide wurden mittels 10% bzw. 20 % denaturierender PAGE gereinigt.

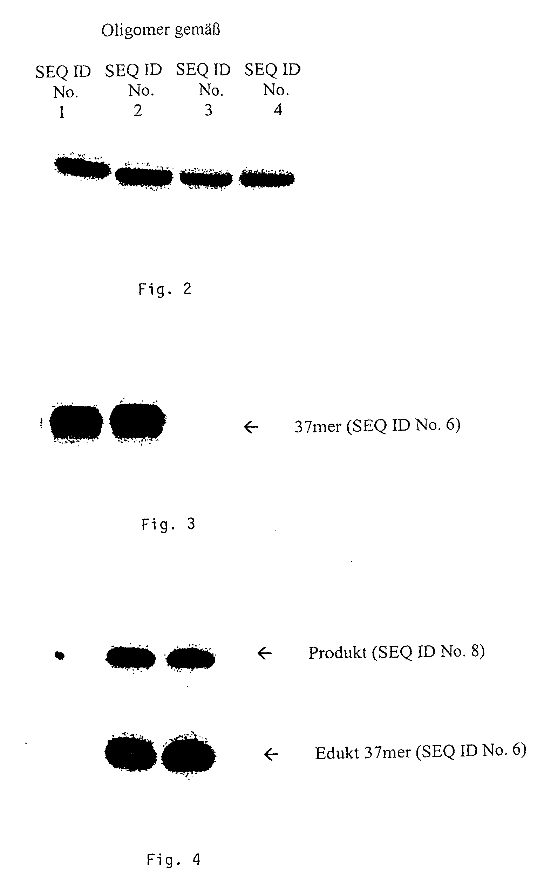

Die 15-mere gemäß SEQ ID No.1-4 (400 pmol) wurden in 5 µl 1x Kinasepuffer (MBI-Fermentas, 50 mM Tris HCl pH 7.5, 10 mM MgCl₂, 5 mM Dithiothreitol, 0.1 mM Spermidin, 0.1 mM EDTA) gelöst und bei 37 °C in Gegenwart von 5 µCi γ-³²P-ATP (3000 Ci/mmol, Hartmann-Analytik) and 40 U T4 Polynucleotidkinase (MBI-Fermentas oder Gibco BRL) inkubiert. Nach 3 Stunden wurde die Reaktionsmischung über eine 20% denaturierende PAGE analysiert. Produkte wurden über Autoradiographie sichtbar gemacht und gegebenenfalls aus dem Gel ausgeschnitten und die Produkte eluiert bzw. die entsprechende Menge an inkorporierter Radioaktivität über Cerenkov-Zählung ermittelt. Das Ergebnis ist in Fig. 2 dargestellt und zeigt das Autoradiogramm eines 20% denaturierenden Polyacrylamidgeles der kinasierten, bzw. 32P-markierten Produkte von SEQ. ID No.1-4

Das 37-mer **6** (400 pmol), entsprechend SEQ. ID. No. 6 und dem ersten Teil der zu markeirenden L-Nukleinsäure, wurde in 5 µl 1x kinase buffer (MBI-Fermentas, 50 mM Tris HCl pH 7.5, 10 mM MgCl₂, 5 mM Dithiothreitol, 0.1 mM Spermidin, 0.1 mM EDTA) gelöst und bei 37 °C in Gegenwart von 80 µCi γ-³²P-ATP (3000 Ci/mmol, Hartmann-Analytik) and 40 U T4 Polynukleotidkinase (MBI-Fermentas oder Gibco BRL) inkubiert. Nach 3 Stunden wurde die Reaktionsmischung über eine 10% denaturierende PAGE analysiert. Produkte wurden über Autoradiographie sichtbar gemacht, aus dem Gel ausgeschnitten und die Produkte eluiert ( in 0.3 M Natriumacetatlösung, 1h schütteln bei 65°C) bzw. die entsprechende Menge an inkorporierter Radioaktivität über Cerenkov-Zählung ermittelt. Das Ergebnis ist in Fig. 2 dargestellt als Autoradiogramm eines 10% denaturierenden Polyacrylamidgeles der kinasierten, bzw. 32P-markierten Produkte von SEQ. ID No.6.

### Beispiel 2: Chemische Ligation

Der teilweise phosphorylierte Strang **6** (entsprechend SEQ ID No.6 und dem ersten Teil der zu markierenden L-Nukleinsäure) (500 pmol), Strang **5** (entsprechend SEQ ID No.5 und dem zweiten Teil der zu markierenden L-Nukleinsäure) (800 pmol) und Matrix **7** (entsprechend SEQ ID No.7) (800 pmol) wurden gelöst in Morpholinoethansulfonsäure-Triethylamin Puffer ( MES TEA) (18 µl 0.55 M, pH 7.75) , der auch 55 mM MgCl₂ enthält, und denaturiert bei 98° C für 4 min.

Nach langsamen Abkühlen (10 min) wurde die Reaktion durch Zugabe von 2 µl 5 M BrCN in DMF bei 0° gestartet.

Nach 15 min bei 0°C wurde NaOH zur Mischung zugefügt (Endkonzentration 0.5 M) und bei 98°C für 10 min lang inkubiert, um die Matrix zu hydrolysieren. Die Neutralisierung wurde durch Hinzufügen des gleichen Volumens an 0.5 M HCl erreicht. Die Reaktionsmischung wurde unter Zugabe von Natriumacetatlösung (pH 5.5, Endkonzentration 0.3M) mit Ethanol gefällt und anschließend über 10% denaturierende PAGE gereinigt. Das Ergebnis ist in Fig. 3 dargestellt. Es zeigt sich deutlich, dass ausgehend von markierten 37-mer gemäß SEQ ID No.6 das ebenfalls markierte 67-mer gemäß SEQ ID No.8 darstellbar ist, welches die im Rahmen dese Beispiels zu markierende L-Nukleinsäure darstellt.

Bei dem Spiegelmer gemäß SEQ ID No.8 handelt es sich im übrigen um ein GnRH-bindendes L-DNA-Oligonukleotid mit der folgenden Sequenz: 
5'-CCA AGC TTG CGT AAG CAG TCT CCT CTC AGG GGA GGT TGG GCG GTG CGT AAG
CAC CGG TTT GCA GGG G -3' (SEQ.ID No.8)
 die sich aus dem Ligieren des ersten Teils entsprechend SEQ.ID No. 6 mit dem zweiten Teil entsprechend SEQ. ID No. 5 der zu markierenden Sequenz (d.h der L-Nukleinsäure gemäß SEQ.ID.No.8) ergibt. Die radioaktive Markierung in Form von ³²P ist zwischen den Positionen 30 und 31 enthalten.

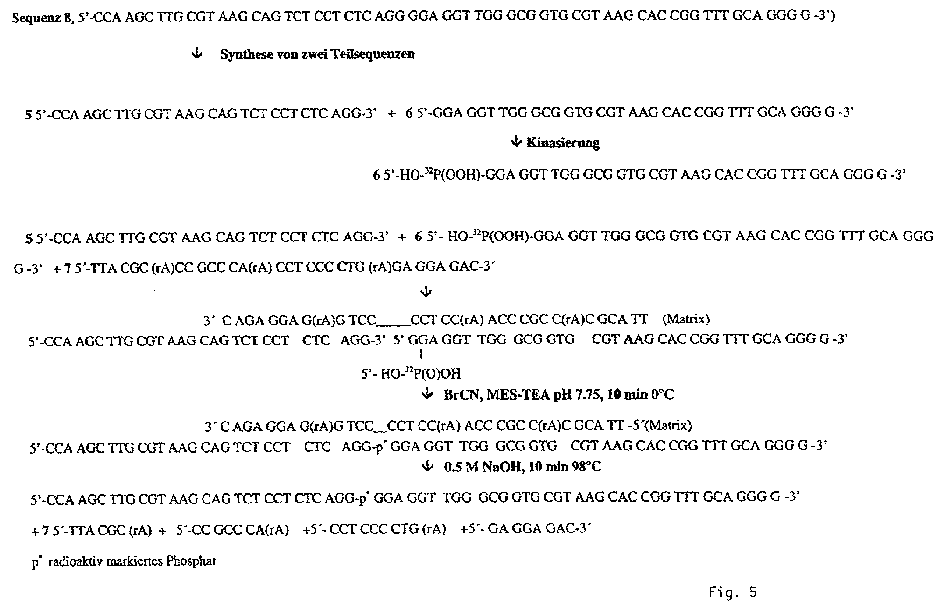

Die vorstehend beschriebene Ligation ist schematisch nochmals in Fig. 5 dargestellt, wobei hier die Teilsequenzen, die terminale Markierung des 5'-Endes der Sequenz 6 sowie die anschließende Ligation and der Matrix und Hydrolyse der Matrix dargestellt ist.

### Beispiel 3: Selektivität der T4-Polynukleotidkinase

Der Einfluß auf das 5'terminale Nukleotid wurde systematisch untersucht. Dabei weisen C und G die besten Kinasierungsausbeuten auf, A und T sind hierbei zwar vergleichsweise geringer phosphoryliert, stellen aber keine ausgeschlossenen Phosphorylierungsstellen dar. Die Ergebnisse sind in den nachfolgenden Tabellen 1 und 2 dargestellt.

In der Literatur sind vergleichende Studien an D-DNA durchgeführt worden, mit dem Ergebnis, dass G das zu präferierende 5'Nucleotid ist (van Houten V, Denkers F, van Dijk M, van den Brekel M, Brakenhoff R. (1998) Labeling efficiency of oligonucleotides by T4 polynucleotide kinase depends on 5'-nucleotide. *Anal Biochem.* **265** ,386-389).

Derart gravierende Unterschiede wurden in der L-Reihe nicht festgestellt. Die hohen Kinasierungsausbeuten der 15mere **1-4** sind durchaus vergleichbar mit denen der natürlichen DNA (van Houten V, Denkers F, van Dijk M, van den Brekel M, Brakenhoff R. (1998) Labeling efficiency of oligonucleotides by T4 polynucleotide kinase depends on 5'nucleotide. *Anal Biochem.* **265**, 386-389)

**Tabelle 1:**

| Kinasierungsausbeute für die Spiegelmere **1-4** | | | | |
|---|---|---|---|---|
| Terminalnucleotid | G | A | C | T |
| Oligo Nr. | **1** | **3** | **2** | **4** |
| Kinasierungsausbeute in % | 43-44 | 26-28 | 47-49 | 30-31 |

Das in Tabelle 1 dargestellt Ergebnis belegt die hierin getroffene Aussage, dass 5'-terminal angeordnete Guanosin- und Cytosin-Reste bevorzugte Substrate der T4-Polynukleotidkinase darstellen.

**Tabelle 2:**

| Kinasierungsausbeute für die Spiegelmere **6** und dessen Enantiomer **D-6** | | |
|---|---|---|
| Konformation | L | D |
| Kinasierungsausbeute | 10-15 | 40-50 |

Wie aus Tabelle 2 ersichtlich, sind bei dem zur Ligation einzusetzenden Strang die Ausbeuten der Kinasierung zwischen D und L durchaus unterschiedlich. Es ist dabei anzuerkennen, dass die aktuellen Ausbeuten von der Art und der Länge der Spiegelmere abhängig sind.

Die in der vorstehenden Beschreibung, den Ansprüchen sowie den, Zeichnungen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Verfahren zum Markieren von L-Nukleinsäuren umfassend die Schritte
a) Bereitstellen eines ersten Teils der zu markierenden L-Nukleinsäure,
b) Markieren des ersten Teils mit einem Marker,
c) Bereitstellen eines zweiten Teils der zu markierenden L-Nukleinsäure, und
d) Ligation des in Schritt b) markierten ersten Teils mit dem zweiten Teil der zu markierenden Nukleinsäure.

2. Verfahren nach Anspruch 1, **dadurch gekennzeichnet, dass** der Marker ausgewählt ist aus der Gruppe, die ³H, ¹³C, ¹⁴C, ¹⁵N, ¹⁸O, ³²P, ³³P und ³⁵S umfasst.

3. Verfahren nach Anspruch 1 oder 2, **dadurch gekennzeichnet, dass** das Markieren des ersten Teils mit einem Marker am 5'-Ende des ersten Teils erfolgt.

4. Verfahren nach Anspruch 3, **dadurch gekennzeichnet, dass** das Markieren durch Umsetzen des ersten Teils mit einer Kinase und einem einen Marker tragenden Nukleotid erfolgt.

5. Verfahren nach Anspruch 4, **dadurch gekennzeichnet, dass** die Kinase T⁴-Polynukleotid-Kinase ist.

6. Verfahren nach Anspruch 4 oder 5, **dadurch gekennzeichnet, dass** das Nukleotid ausgewählt ist aus der Gruppe, die γ-[¹⁸O]-ATP, γ-[³²P]-ATP, γ-[³³P]-ATP ,γ-[³⁵S]-ATP und Adenosinphosphonate und Derivate davon umfasst.

7. Verfahren nach einem der Ansprüche 1 bis 6, **dadurch gekennzeichnet, dass** die Ligation durch Verknüpfen des 5'-Endes des ersten Teils mit dem 3'-Ende des zweiten Teils erfolgt.

8. Verfahren nach einem der Ansprüche 1 bis 7, **dadurch gekennzeichnet, dass** die Ligation der beiden Teile an einer zumindest teilweisen komplementären Matrix erfolgt.

9. Verfahren nach einem der Ansprüche 1 bis 8, **dadurch gekennzeichnet, dass** die Ligation unter Verwendung eines Kopplungsreagenz erfolgt, wobei das Kopplungsreagenz ausgewählt ist aus der Gruppe, die BrCN und Imidazole und Imidazolderivate umfasst.

10. Verfahren nach einem der Ansprüche 1 bis 9, **dadurch gekennzeichnet, dass** die L-Nukleinsäure ein Spiegelmer ist.

11. L-Nukleinsäure umfassend eine Markierung, wobei die Markierung an einem nicht-terminalen Nukleotid der Nukleinsäure angeordnet ist.

12. L-Nukleinsäure nach Anspruch 11, **dadurch gekennzeichnet, dass** die Markierung eine radioaktive Markierung ist.

13. L-Nukleinsäure nach Anspruch 12, **dadurch gekennzeichnet, dass** die Markierung ausgewählt ist aus der Gruppe, die ³H, ¹³C, ¹⁴C, ¹⁵N, ¹⁸O, ³²P, ³³P und ³⁵S umfasst.

14. L-Nukleinsäure nach einem der Ansprüche 11 bis 13, **dadurch gekennzeichnet, dass** zwischen zwei aufeinanderfolgenden Guanidin-Nukleosiden ein radioaktives Nuklid als Marker angeordnet ist.

15. L-Nukleinsäure nach einem der Ansprüche 11 bis 14, **dadurch gekennzeichnet, dass** die Nukleinsäure ein Spiegelmer ist und bevorzugterweise eine Sequenz gemäß SEQ ID NO:8 aufweist.

16. L-Nukleinsäure nach Anspruch 15, **dadurch gekennzeichnet, dass** das Phosphat zwischen Position 30 und 31 von SEQ ID NO:8 ³²P ist.

17. Verwendung einer T4-Polynukleotidkinase zum Markieren einer L-Nukleinsäure.

18. Verwendung nach Anspruch 17, **dadurch gekennzeichnet, dass** die Markierung innerhalb der L-Nukleinsäure erfolgt.

19. Verwendung nach Anspruch 17, **dadurch gekennzeichnet, dass** die Markierung terminal an der L-Nukleinsäure erfolgt.

20. Verwendung einer Nukleinsäure nach einem der Ansprüche 11 bis 16 zum Nachweis einer Verbindung, an die die Nukleinsäure bindet, wobei die Verbindung in einer biologischen Probe enthalten ist.

21. Verwendung einer Nukleinsäure nach einem der Ansprüche 11 bis 16 zur Herstellung eines Medikamentes.

22. Verwendung einer Nukleinsäure nach einem der Ansprüche 11 bis 16 zur Herstellung eines diagnostischen Mittels.

23. Verwendung nach Anspruch 22, **dadurch gekennzeichnet, dass** die Nukleinsäure ein Spiegelmer ist, wobei das Spiegelmer gegen ein Zielmolekül gerichtet ist.

24. Verwendung nach Anspruch 23, **dadurch gekennzeichnet, dass** das Zielmolekül an einem pathogenen Prozess beteiligt ist oder ein Marker für einen pathogenen Prozess darstellt.


SEQUENZPROTOKOLL

<110> NOXXON Pharma AG

<120> Verfahren zur Markierung von L-Nukleinsäuren

<130> N 10017 EP

<140>
<141>

<160> 8

<170> PatentIn Ver. 2.1

<210> 1
<211> 14
<212> DNA
<213> Künstliche Sequenz

<220>
<223> Beschreibung der künstlichen Sequenz:
      Oligonukleotid

<400> 1
ggattacagc gctg                                                    14


<210> 2
<211> 15
<212> DNA
<213> Künstliche Sequenz

<220>
<223> Beschreibung der künstlichen Sequenz:
      Oligonukleotid

<400> 2
cgagttacag cgctg                                                   15


<210> 3
<211> 15
<212> DNA
<213> Künstliche Sequenz

<220>
<223> Beschreibung der künstlichen Sequenz:
      Oligonukleotid

<400> 3
agagttacag cgctg                                                   15


<210> 4
<211> 15
<212> DNA
<213> Künstliche Sequenz

<220>
<223> Beschreibung der künstlichen Sequenz:
      Oligonukleotid

<400> 4
tgagttacag cgctg                                                        15


<210> 5
<211> 30
<212> DNA
<213> Künstliche Sequenz

<220>
<223> Beschreibung der künstlichen Sequenz:
      Oligonukleotid

<400> 5
ccaagcttgc gtaagcagtc tcctctcagg                                        30


<210> 6
<211> 37
<212> DNA
<213> Künstliche Sequenz

<220>
<223> Beschreibung der künstlichen Sequenz:
      Oligonukleotid

<400> 6
ggaggttggg cggtgcgtaa gcaccggttt gcagggg                                37


<210> 7
<211> 33
<212> DNA
<213> Künstliche Sequenz

<220>
<223> Beschreibung der künstlichen Sequenz:
      Oligonukleotid

<220>
<223> Ribonukleotid statt Desoxyribonukleotid an den
      Positionen 7, 15 und 25

<400> 7
ttacgcaccg cccaacctcc cctgagagga gac                                    33


<210> 8
<211> 67
<212> DNA
<213> Künstliche Sequenz

<220>
<223> Beschreibung der künstlichen Sequenz:
      GnRH-bindendes L-DNA-Oligonukleotid

<400> 8
ccaagcttgc gtaagcagtc tcctctcagg ggaggttggg cggtgcgtaa gcaccggttt 60
gcagggg                                                                 67